# EUROPEAN PATENT APPLICATION

(11) **EP 4 403 091 A1**
(43) Date of publication of application: **24.07.2024**
(21) Application number: 24152631.8
(22) Date of filing: 18.01.2024
(51) Int. Cl.: A61B 3/00, A61B 3/15

(54) **OPHTHALMOLOGIC APPARATUS**

(30) Priority: 23.01.2023 JP 2023008200
(71) Applicant: TOPCON CORPORATION, Tokyo 174-8580 (JP)
(72) Inventor: UJIIE, Takumi, Tokyo, 174-8580 (JP); OOHARA, Ryuichi, Tokyo, 174-8580 (JP)
(74) Representative: Louis Pöhlau Lohrentz

(57) **Abstract**

An ophthalmologic apparatus (A) includes a main body (20) including a measurement optical system (50) that is configured to measure eye characteristics of a subject eye (E) of a subject in a state where the subject has placed his or her chin on a chin rest (30); an anterior ocular segment camera (22) that is provided in the main body (20), the anterior ocular segment camera (22) being configured to acquire an anterior ocular segment image by imaging an anterior ocular segment of the subject; and a controller (60) that is configured to control each element of the apparatus. The controller (60) comprises an alignment controller (63) that is configured to perform control to adjust a relative positional relationship between the subject eye (E) and the main body (20) based on the anterior ocular segment image. The alignment controller (63) includes an imaging start mode controller (631). The imaging start mode controller (631) is configured, when it is determined that the subject eye (E) does not appear in the anterior ocular segment image in an imaging start mode of the anterior ocular segment by the anterior ocular segment camera (22), to calculate a predicted position of the subject eye (E) based on image recognition of a face part of the subject in the anterior ocular segment image automatically following the determination or automatically after performing at least notification following the determination, and to perform control of movement toward the calculated predicted position of the subject eye (E).

## Description

### FIELD OF THE INVENTION

The present invention relates to an ophthalmologic apparatus.

### BACKGROUND

An ophthalmologic apparatus includes a magnification changing means that changes the magnification at which an image of a face of a subject including an external eye area of a subject eye is observed by an observation means. There is a known ophthalmologic apparatus in which, when it is detected that the subject eye is on an optical axis of a measurement means, the magnification changing means sets an image to be given to the observation means as a high magnification image, and when it is detected that the subject eye is not on the optical axis of the measurement means, the magnification changing means sets an image to be given to the observation means as a low magnification image (see Patent Literature 1: JPH08-224213A).

In an ophthalmologic apparatus for examining a subject eye, a region of interest based on the position of an optometry portion moved by an adjustment means is set in a captured image. An arithmetic processing portion that processes an image signal in the region of interest controls an imaging condition of a face imaging means based on the image signal in the region of interest. An ophthalmologic apparatus is known in which movement of an optometry portion by an adjustment means is controlled based on a captured image acquired under a controlled imaging condition (see Patent Literature 2: JP6843527B).

An information acquisition portion that acquires information of a position regarding at least one of the subject eye or an optical head portion as output data from a learned model by using an image regarding the subject eye acquired using the optical head portion as input data of the learned model is provided. There is a known ophthalmologic apparatus in which a drive control portion controls the driving of at least one of a support portion and an optical head portion based on acquired information of a position to move at least one of the subject eye or the optical head portion to the position (see Patent Literature 3: JP7194136B).

Meanwhile, when eye characteristics of the subject eye are measured in a state where a chin of the subject is supported on a chin rest, alignment control that is adjustment control of the relative positional relationship between the subject eye and a main body is performed on the condition that the subject eye appears in an anterior ocular segment image when imaging of the anterior ocular segment is started. However, in situations such as when the face of the subject is not facing the front and inclined, when the chin of the subject is not correctly placed on the chin rest, when the initial height position of the chin rest is shifted due to an age difference from the previous subject, or the like, the subject's eye may not appear in the anterior ocular segment image. Therefore, when the subject eye does not appear in the anterior ocular segment image in an imaging start mode, it is necessary to perform preparation work for making the subject eye appear in the anterior ocular segment image. The preparation work includes correction of the inclination of the face of the examinee by the examiner, alignment adjustment by manual operation by the examiner, or the like.

In particular, when the examiner measures the eye characteristics by remote control at a position away from the subject or in another room, visual confirmation of the relative positional relationship between the subject eye and the main body by the examiner or handling of manual operation by the examiner is difficult. Thus, when the subject eye does not appear in the anterior ocular segment image at the time of the remote operation, the examiner needs to move to the position of the examinee to perform the preparation work, which requires man-hours including the movement of the position of the examiner.

On the other hand, in the technology disclosed in Patent Literature 1, the magnification changing means is an essential element, and the problem cannot be solved in the imaging start mode without magnification changing means. Patent Literature 2 discloses a technique of detecting the position of a subject eye appearing in an anterior ocular segment image from a face imaging portion, determining whether or not the subject eye has been detected in the anterior ocular segment image from an anterior ocular segment imaging optical system, and performing alignment control. Patent Literature 3 discloses a technique of acquiring the position information of at least one of a subject eye or an optical head portion using an image regarding the subject eye acquired using the optical head portion and a learned model, and performing alignment control. However, the techniques disclosed in Patent Literatures 2 and 3 are both alignment techniques on the condition that the subject eye appears in the anterior ocular segment image, and thus the above problem in the case where the subject eye does not appear cannot be solved.

The present invention has been made in view of the above problems, and an object of the present invention is to provide an ophthalmologic apparatus that automatically performs alignment adjustment to a position where a subject eye appears when the subject eye does not appear in an anterior ocular segment image in an imaging start mode of an anterior ocular segment.

### SUMMARY

To achieve the above object, an ophthalmologic apparatus includes a main body including a measurement optical system that is configured to measure eye characteristics of a subject eye of a subject in a state where the subject has placed his or her chin on a chin rest; an anterior ocular segment camera that is provided in the main body, the anterior ocular segment camera being configured to acquire an anterior ocular segment image by imaging an anterior ocular segment of the subject; and a controller that is configured to control each element of the apparatus. The controller includes an alignment controller that is configured to perform control to adjust a relative positional relationship between the subject eye and the main body based on the anterior ocular segment image. The alignment controller includes an imaging start mode controller. The imaging start mode controller is configured, when it is determined that the subject eye does not appear in the anterior ocular segment image in an imaging start mode of the anterior ocular segment by the anterior ocular segment camera, to calculate a predicted position of the subject eye based on image recognition of a face part of the subject appearing in the anterior ocular segment image automatically following the determination or automatically after performing at least notification following the determination, and to perform control of movement toward the calculated predicted position of the subject eye.

In the ophthalmologic apparatus of the present invention, alignment adjustment to a position where the subject eye appears can be automatically performed when the subject eye does not appear in the anterior ocular segment image in the imaging start mode of the anterior ocular segment,

### BRIEF DESCRIPTION OF DRAWINGS

FIG. 1 is a perspective view illustrating an external configuration of an ophthalmologic apparatus of a first embodiment as viewed obliquely from a side where a chin rest is positioned.
FIG. 2 is a perspective view illustrating the external configuration of the ophthalmologic apparatus of the first embodiment as viewed obliquely from a side where a control panel is positioned.
FIG. 3 is a front view illustrating the external configuration of the ophthalmologic apparatus of the first embodiment when the main body is viewed from the side where the chin rest is positioned.
FIG. 4 is a side view illustrating a schematic configuration of incorporated elements and accessories of the ophthalmologic apparatus of the first embodiment.
FIG. 5 is a block diagram illustrating a configuration of a control system in the ophthalmologic apparatus of the first embodiment.
FIG. 6 is a flowchart illustrating a flow of alignment control processing in an imaging start mode controller of the first embodiment.
FIG. 7 is a view illustrating an example of an imaging start mode alignment image displayed on a display screen of the control panel at the time of imaging start mode control.
FIG. 8 is an explanatory diagram illustrating an image movement example by calculation of a predicted position of a subject eye from an imaging start mode alignment image in which the subject eye does not appear and a low magnification image change example when a feature portion of a face is not detected.
FIG. 9 is an explanatory diagram illustrating a detection example of a feature portion of the face of a subject in the imaging start mode alignment image in which a subject eye does not appear.
FIG. 10 is a diagram illustrating an example of an alignment image of a pupil height position displayed on the display screen of the control panel at a time of coarse alignment control.
FIG. 11 is a view illustrating an example of an auto-alignment image of a pupil position displayed on the display screen of the control panel at a time of fine alignment control.
FIG. 12 is a view illustrating an example of a manual alignment image of a pupil position displayed on the display screen of the control panel at the time of the fine alignment control.
FIG. 13 is a flowchart illustrating a flow of alignment control processing in an imaging start mode controller of a second embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for the sake of clarity.

Embodiments for implementing an ophthalmologic apparatus according to the present invention will be described based on first and second embodiments illustrated in the drawings. The first and second embodiments are application examples to an ophthalmologic apparatus that observes, captures or images, and records an anterior ocular segment image of a subject eye, a fundus image of the subject eye, and a fundus tomographic image of the subject eye, and provides the images as electronic images for diagnosis. Note that in each drawing, an X-axis represents a left-right axis extending in a left-right direction (horizontal direction) when the subject eye faces the main body of the ophthalmologic apparatus, a Y-axis represents an up-down axis extending in an up-down direction (vertical direction), and a Z-axis represents a front-back axis extending in a front-back direction (depth direction) orthogonal to the X-axis and the Y-axis.

### First Embodiment

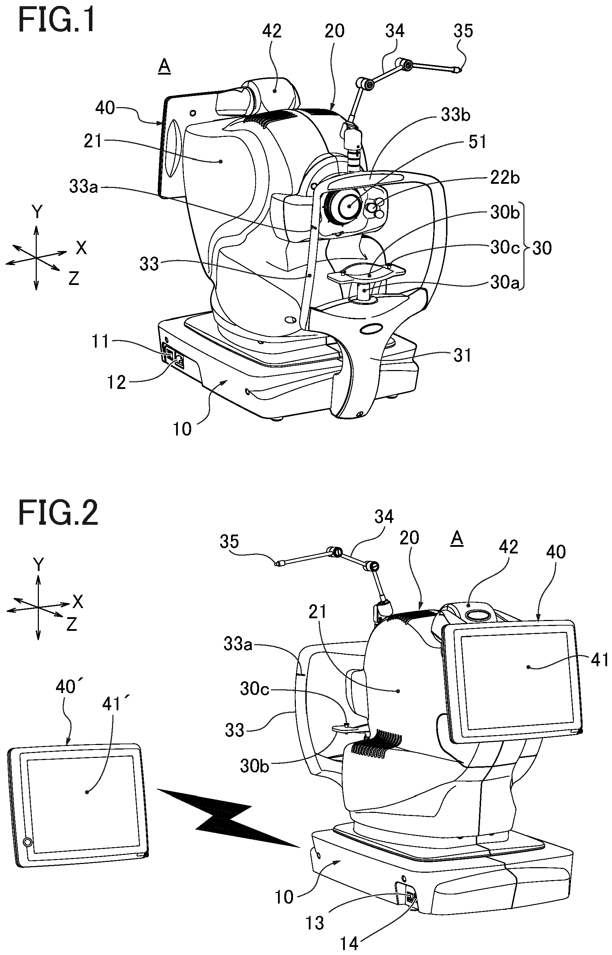

An overall apparatus configuration of an ophthalmologic apparatus A is shown in FIGS. 1 to 4. The ophthalmologic apparatus A is referred to as a "three-dimensional fundus imaging apparatus". As illustrated in FIGS. 1 to 4, the ophthalmologic apparatus A includes a stand 10, a main body 20, a chin rest 30, a control panel 40, a measurement optical system 50, and a controller 60.

The ophthalmologic apparatus A includes a fundus camera that acquires a fundus image of a subject eye E, and an OCT (Optical Coherence Tomography) that acquires a fundus tomographic image of the subject eye E. Here, the "fundus camera" refers to a camera that images a fundus state of a retina, an optic nerve, a capillary blood vessel, and the like at the back of the subject eye E and captures a fundus image. The "OCT" refers to an optical coherence tomography that images a tomographic image of a retina existing in the fundus of the subject eye E using light interference, and captures a fundus tomographic image.

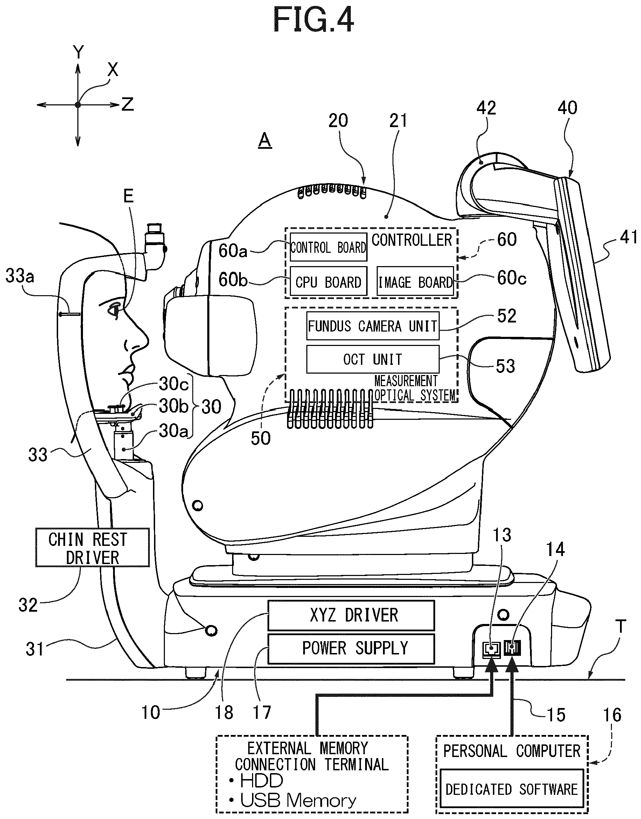

The stand 10 is placed on a table such as an optometry table T (not illustrated) whose height can be adjusted. At an upper surface position of the stand 10, the main body 20 is supported movably in three axial directions of an X-axis, a Y-axis, and a Z-axis. The chin rest 30 is fixed to a front surface position of the stand 10. A power switch 11, a power inlet 12, a USB terminal 13, and a LAN terminal 14 are provided at side surface positions of the stand 10. Note that USB is an abbreviation for "Universal Serial Bus", and LAN is an abbreviation for "Local Area Network". The USB terminal 13 is an external memory connection terminal to which a hard disk drive (HDD) (Hard Disk Drive), USB memory, and the like are connected as illustrated in FIG. 4. The LAN terminal 14 is connected to a personal computer 16 in which dedicated software or the like is installed via a LAN cable 15.

As illustrated in FIG. 4, a power supply 17 and an XYZ driver 18 are provided within an internal space of the stand 10. The power supply 17 includes the power switch 11, the power inlet 12, the USB terminal 13, the LAN terminal 14, and the like. The XYZ driver 18 is a motor actuator including a motor and a motor drive circuit that drive the main body 20 in three axial directions of the X, Y, and Z axes when the main body 20 is moved with respect to the stand 10 in the alignment control.

The main body 20 is provided to be movable in an X-axis direction, a Y-axis direction, and a Z-axis direction by the XYZ driver 18 with respect to the stand 10 to which the chin rest 30 is fixed. In the main body 20, the measurement optical system 50 is incorporated in a main body cover 21 that covers the entire main body. The measurement optical system 50 measures eye characteristics of the subject eye E in a state where the chin of a subject is supported on the chin rest 30. As illustrated in FIGS. 1, 2, and 4, the control panel 40 is disposed at an upper position of a back surface of the main body cover 21. As illustrated in FIG. 4, the controller 60 is provided within the internal space of the main body cover 21 in addition to the measurement optical system 50.

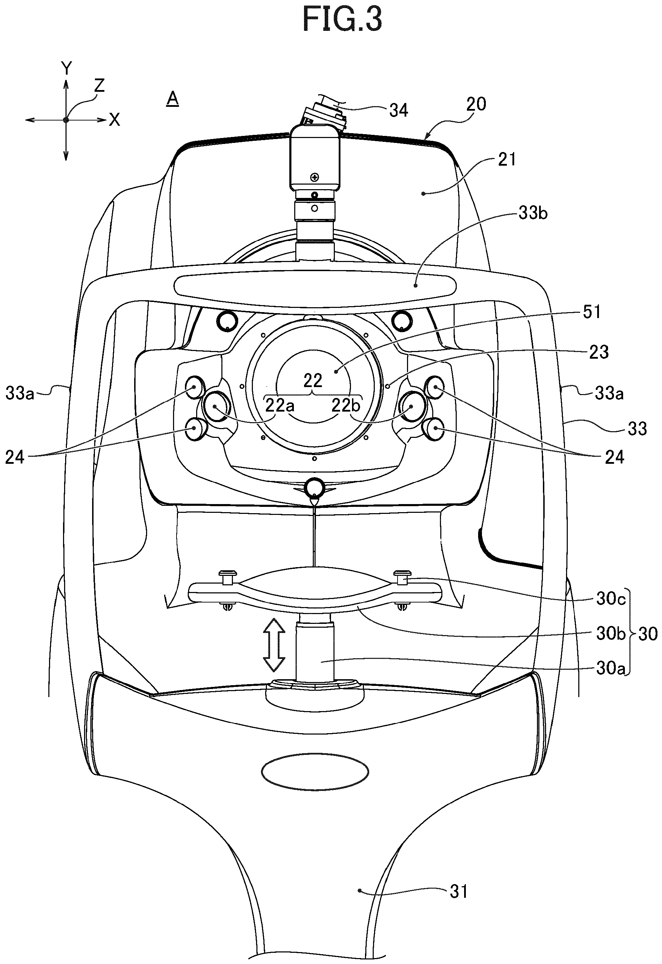

As illustrated in FIG. 3, the objective lens 51 of the measurement optical system 50 is provided in the center of the front surface position of the main body cover 21 to face the subject eye E. Around the objective lens 51, an anterior ocular segment stereo camera 22 (anterior ocular segment camera), a peripheral fixation lamp 23, and an anterior ocular segment observation filter 24 are provided.

The anterior ocular segment stereo camera 22 is a camera that acquires an anterior ocular segment image by capturing the anterior ocular segment of the subject. The anterior ocular segment stereo camera 22 includes a first camera 22a and a second camera 22b at both sides of the objective lens 51. In the first and second cameras 22a, 22b, lens optical axes are arranged to be inclined toward the anterior ocular segment of the subject eye E to be measured. The first and second cameras 22a, 22b are magnification cameras capable of switching between high magnification and low magnification and stepless magnification change. The first and second cameras 22a, 22b acquire a right anterior ocular segment image and a left anterior ocular segment image obtained by cutting a part of the face of the subject supported by the chin rest 30 according to the selection of the subject eye E and the angle of view at that time. The first camera 22a and the second camera 22b of the anterior ocular segment stereo camera 22 are arranged by determining the inclination angle and the width dimension in the X-axis direction, and accordingly, the three-dimensional coordinate position of the subject eye E can be specified by calculation processing based on the two anterior ocular segment images.

The peripheral fixation lamps 23 are fixation lamps used to fix the line of sight of the subject eye E by turning on. Eight peripheral fixation lamps 23 are arranged at equal intervals at outer peripheral positions of the objective lens 51. The anterior ocular segment observation filter 24 is a filter used to adjust the amount of light in anterior ocular segment observation or the anterior ocular segment OCT. Two anterior ocular segment observation filters 24 are arranged in each of longitudinal directions of the outer position of the first camera 22a and the outer position of the second camera 22b. This provides a total of four anterior ocular segment observation filters 24.

The chin rest 30 is provided to be adjustable in height position (position in the Y-axis direction) with respect to a chin rest support portion 31 fixed to the stand 10 to support the chin of the subject. The chin rest 30 includes a lifting rod 30a, a chin rest base 30b, and chin rest sheet fixing pins 30c. The lifting rod 30a is lifted and lowered by an incorporated chin rest driver 32. The chin rest base 30b is fixed to an upper end of the lifting rod 30a. The chin rest sheet fixing pins 30c are provided at both sides of the chin rest base 30b. The chin rest driver 32 is a motor actuator including a motor drive circuit and a motor that drives the lifting rod 30a in the Y-axis direction when the chin rest 30 is moved in the Y-axis direction with respect to the chin rest support portion 31 and the stand 10 in the alignment control.

The chin rest support portion 31 is T-shaped and a face support frame 33 is fixed to the chin rest support portion 31 at both ends of the T-shaped chin rest support portion 31. The face support frame 33 includes a pair of vertical frames and a horizontal frame that connects the upper ends of the vertical frames. The face support frame 33 has a shape enclosing the face of the subject in three directions when the chin of the subject is supported by the chin rest 30. Each of the vertical frames extending in the Y-axis direction is provided with a height mark 33a that functions as a reference of the height position of the subject eye E. The horizontal frame of the face support frame 33 is provided with a detachable forehead contact surface 33b formed of silicone rubber or the like. Further, the horizontal frame is provided with an arm 34 at a central upper position thereof. The arm 34 is configured to be bent in multiple steps and an external fixation target 35 is provided at a distal end of the arm 34.

The control panel 40 is disposed at the upper position of the back surface of the main body cover 21. The control panel 40 includes a display screen 41 that displays an anterior ocular segment image of the subject eye E from the anterior ocular segment stereo camera 22, an anterior ocular segment observation image of the subject eye E from the measurement optical system 50, and the like in color. The display screen 41 is a touch panel on which the examiner performs an input operation to the controller 60 by touching a displayed button image, image, or the like with a finger. The coupling support portion 42 of the control panel 40 with respect to the main body 20 has a combination support structure of bending support and rotation support in which the display screen 41 can be set at any position in the entire circumferential direction with respect to the main body 20 and the inclination angle of the display screen 41 can be freely set. That is, the control panel 40 included in the main body 20 is used when the examiner stays close to the subject to examine the eye characteristics. For this reason, the coupling support portion 42 ensures a function that the display screen 41 can be arranged in a position where the examiner can easily operate the display screen 41 at any position around the ophthalmologic apparatus A.

When the examiner performs the examination of the eye characteristics by remote control from a position away from the subject, for example, as illustrated in FIG. 2, a remote control tablet 40' having a communication function with the main body 20 in addition to an input operation function equivalent to the control panel 40 is used. Thus, the remote control tablet 40' also has a display screen 41' by a touch panel.

The measurement optical system 50 measures the eye characteristics of the subject eye E in a state where the chin of the subject is supported on the chin rest 30, and includes a fundus camera unit 52 having the objective lens 51 and an OCT unit 53 as illustrated in FIG. 4. The fundus camera unit 52 includes an illumination optical system and an imaging optical system and constitutes a fundus camera that acquires a fundus image of the subject eye E by a lens, an imaging element, and the like. The OCT unit 53 constitutes an OCT that acquires a fundus tomographic image of the subject eye E by a wavelength-tunable light source, a fiber coupler, or the like. Note that an anterior ocular segment observation image of the subject eye E can be acquired from the measurement optical system 50 in addition to the fundus image and the fundus tomographic image of the subject eye E.

The controller 60 controls the elements of the apparatus such as the fundus camera unit 52, the OCT unit 53, the chin rest 30, the main body 20, and the like based on various input operations including a touch operation on the display screen 41 of the control panel 40. As illustrated in FIG. 4, the controller 60 includes a control board 60a, a CPU board 60b, and an image board 60c as a hardware configuration.

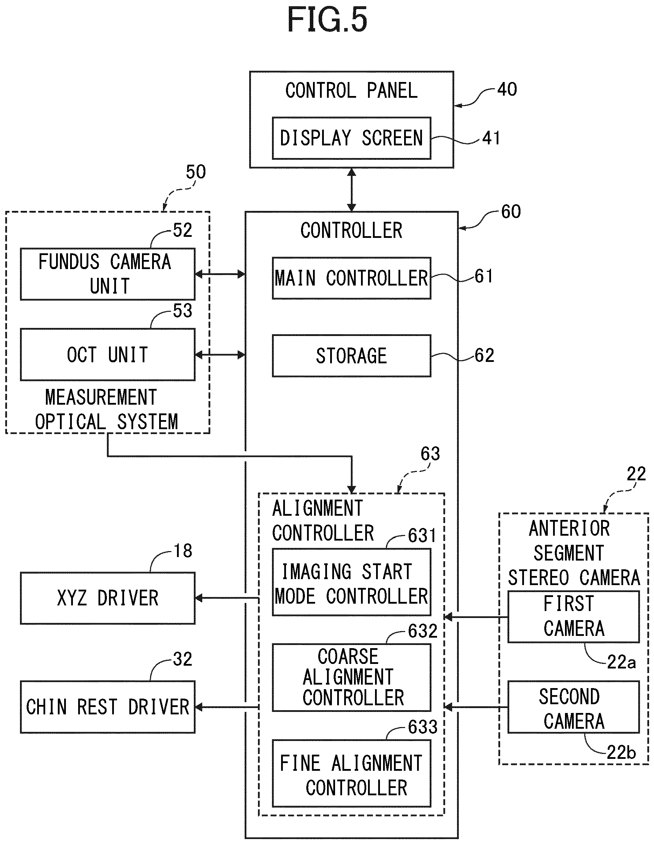

(Control system configuration) As illustrated in FIG. 5, a control system configuration of the ophthalmologic apparatus A includes the control panel 40 (display 41), the measurement optical system 50 (fundus camera unit 52 and OCT unit 53), and the controller 60.

The controller 60 includes a main controller 61 that controls the fundus camera unit 52 and the OCT unit 53, a storage 62 that stores necessary data, and an alignment controller 63. The alignment controller 63 performs alignment control to adjust the relative positional relationship between the subject eye E and the main body 20 (the objective lens 51 included in the main body 20) based on the anterior ocular segment image acquired by the anterior ocular segment stereo camera 22. The alignment controller 63 includes an imaging start mode controller 631, a coarse alignment controller 632, and a fine alignment controller 633. Hereinafter, coarse alignment control and fine alignment control executed when the subject eye appears in the anterior ocular segment image are collectively referred to as "normal alignment control".

The alignment controller 63 acquires an anterior ocular segment image by capturing the left side and the right side of the subject's face from two directions by the anterior ocular segment stereo camera 22 (first camera 22a and second camera 22b) and acquires an anterior ocular segment observation image by capturing the right eye and the left eye by the measurement optical system 50. The alignment controller 63 adjusts the relative positional relationship between the subject eye E and the objective lens 51 in the main body 20 by a drive command which is output to at least one of the XYZ driver 18 or the chin rest driver 32. The XYZ driver 18 and the chin rest driver 32 are selectively used, and the XYZ driver 18 is used if the adjustment movement amount is a movement amount only in the XZ-axis direction. On the other hand, when the adjustment movement amount includes a movement amount in the Y-axis direction, the XYZ driver 18 and the chin rest driver 32 are selectively used since the movement allowable range of the chin rest driver 32 is wider than the movement allowable range of the XYZ driver 18. For example, the coarse alignment control uses the chin rest driver 32 in the Y-axis movement, and the fine alignment control uses the XYZ driver 18.

When it is determined that the subject eye E does not appear in the anterior ocular segment image in the imaging start mode of the anterior ocular segment by the anterior ocular segment stereo camera 22, after the determination, the imaging start mode controller 631 automatically calculates a predicted position of the subject eye E based on image recognition of the face part appearing in the anterior ocular segment image. Then, control is performed to move toward the calculated predicted position of the subject eye E. Furthermore, when the subject eye E is detected in the anterior ocular segment image from the anterior ocular segment stereo camera 22 after starting the control of moving toward the predicted presence position of the subject eye E, the imaging start mode controller 631 stops the movement at the time of the detection and switches the control to the normal alignment control.

In addition, when it is determined that the subject eye E does not appear in the anterior ocular segment image and the presence position of the subject eye E has not been predicted, the imaging start mode controller 631 decreases the magnification of the anterior ocular segment stereo camera 22 and acquires the anterior ocular segment image from the anterior ocular segment stereo camera 22 again. Here, "when the presence position of the subject eye E has not been predicted" in the first embodiment means that a feature portion of the face used for predicting the presence position of the subject eye E is not detected from the anterior ocular segment image.

When it is confirmed that the subject eye E appears in the displayed anterior ocular segment image, the coarse alignment controller 632 roughly performs alignment control so that the position of the pupil of the subject eye E exists near the center of the image. This coarse alignment control is performed by a manual operation by the examiner while viewing the display images of the anterior ocular segment image and the anterior ocular segment observation image.

When it is confirmed that the position of the pupil exists near the center of the display image, the fine alignment controller 633 performs alignment control on the pupil so that the position of the pupil is placed in the center of the image. This fine alignment control is performed by automatic control based on the two anterior ocular segment images from the anterior ocular segment stereo camera 22, but may also be performed by a manual operation according to the selection of the examiner.

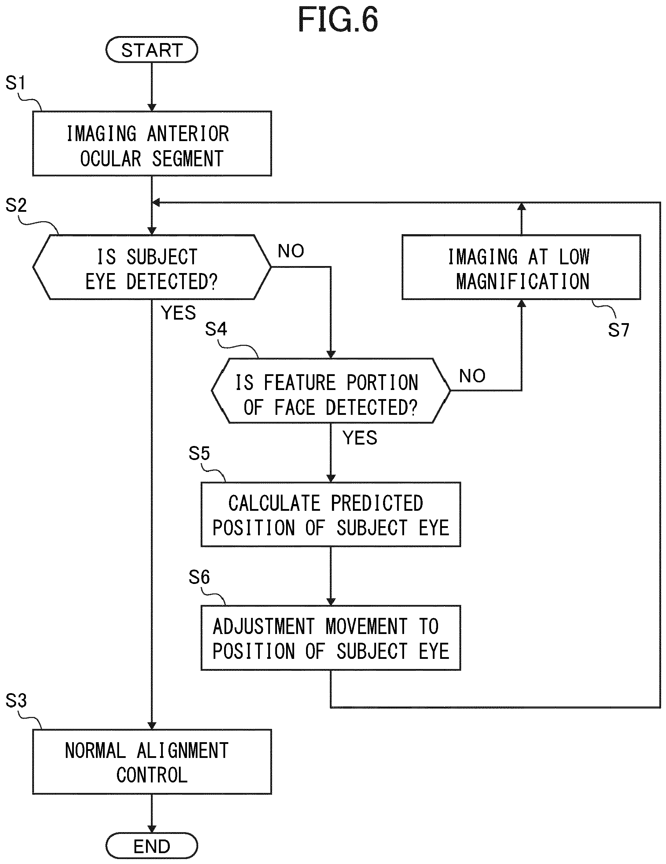

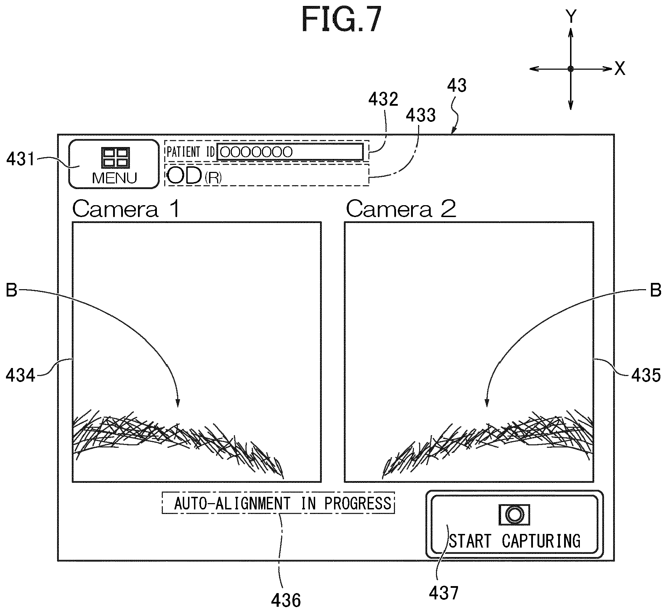

(Processing configuration and processing operation of imaging start mode control) A processing configuration of the imaging start mode control executed by the imaging start mode controller 631 will be described with reference to a flowchart illustrated in FIG. 6. Note that the imaging start mode control process is started by a predetermined operation after it is confirmed that the subject is seated in front of the ophthalmologic apparatus A with the power switch turned on and the chin is supported by the chin rest 30. Here, the "predetermined operation" refers to, for example, an operation of tapping a capture start button 437 on an imaging start auto-adjustment mode screen 43 (see FIG. 7) switched by a tap operation on an imaged eye selection button on an imaging icon selection screen (not illustrated).

In Step S1, following the start, the anterior ocular segment imaging is started by the anterior ocular segment stereo camera 22, and the process proceeds to Step S2. Here, when the processing operation of the imaging start mode control is started, the anterior ocular segment imaging by the anterior ocular segment stereo camera 22 is started, and acquisition of the anterior ocular segment image by a moving image is continued after the imaging of the anterior ocular segment image is started.

In Step S2, following the anterior ocular segment imaging in Step S1, adjustment movement to the subject eye position in Step S6, or low magnification imaging in Step S7, it is determined whether or not the subject eye E is detected in the anterior ocular segment image displayed on the imaging start auto-adjustment mode screen 43. When the subject eye E is detected (i.e., YES) in Step S2, the process proceeds to Step S3. On the other hand, when the subject eye E is not detected (i.e., NO) in Step S2, the process proceeds to Step S4.

Here, the determination as to whether or not the subject eye E has been detected in the anterior ocular segment image is made by determining whether or not the pupil of the subject eye E appears in the anterior ocular segment image. That is, the image processing of converting the anterior ocular segment image into a luminance image indicating the level of luminance is performed, and when a circular pupil having the lowest luminance is detected in the converted luminance image, it is determined that the subject eye E appears in the anterior ocular segment image, and it is determined that the subject eye E is detected in the anterior ocular segment image. On the other hand, when the circular pupil having the lowest luminance is not detected in the luminance image, it is determined that the subject eye E does not appear in the anterior ocular segment image, and it is determined that the subject eye E is not detected in the anterior ocular segment image.

In addition, the determination that the subject eye E is detected in the anterior ocular segment image in Step S2 refers to a determination that the subject eye E (pupil) is detected in the anterior ocular segment images from both the first camera 22a and the second camera 22b. Therefore, even if the subject eye E (pupil) is detected only in the anterior ocular segment image from one of the first camera 22a and the second camera 22b, it is determined in Step S2 that the subject eye E is not detected in the anterior ocular segment image.

In Step S3, following the determination of detection of the subject eye E in Step S2, the normal alignment control, that is, the coarse alignment control is executed and then the fine alignment control is executed, and the process proceeds to the end.

In Step S4, following the determination in Step S2 that the subject eye E is not detected, it is determined whether or not a feature portion of the face is detected by machine learning detection processing using the anterior ocular segment image in which the subject eye E does not appear and a learned feature portion detection model. When the feature portion is detected (i.e., YES) in Step S4, the process proceeds to Step S5. On the other hand, when the feature portion is not detected (i.e., NO) in Step S4, the process proceeds to Step S7. That is, when it is determined that the subject eye E does not appear in the anterior ocular segment image, following the determination, the feature portion of the face extracted from the anterior ocular segment image in which the subject eye E does not appear is automatically detected using the learned feature portion detection model. Here, the "feature portion of the face" refers to a part of the face (for example, an eyebrow, a contour, a nose, a mouth, an ear, or the like) with which the position of the subject eye E can be predicted based on the positional relationship in the entire face image by specifying a partially appearing face part among portions of the face other than the subject eye E by image recognition.

The "learned feature portion detection model" refers to a model constructed in advance by execution of machine learning using a large number of machine learning data sets generated by associating feature portion image data with feature portion information and a selected machine learning model. Here, the "feature portion image data" refers to image data acquired by cutting out a feature portion of the face other than an eye from a large number of pieces of face image data acquired as samples. The "feature portion information" refers to information indicating the name of a feature portion of the face indicated by the acquired image data or a position of the feature portion with respect to the entire face image. The "machine learning model" refers to a model selected from various machine learning algorithm models in accordance with an accuracy requirement level of image recognition for recognizing a feature portion of the face. As the "machine learning model", a "convolutional neural network model" that uses a deep learning method for image recognition and is capable of detecting local features of an image, or the like is selected for example. The "learned feature portion detection model" is constructed in advance by the personal computer 16, for example, and is stored and set to be readable by connection via the LAN cable 15 at the time of the control processing by the imaging start mode controller 631. Note that the learned feature portion detection model may be appropriately updated by changing or adding a machine-learning data set.

In Step S5, following the determination that the feature portion of the face is detected in Step S4, a predicted position of the subject eye E is calculated, and the process proceeds to Step S6. That is, in a case where it is determined that the subject eye E does not appear in the anterior ocular segment image, when the feature portion of the face is detected from the anterior ocular segment image in which the subject eye E does not appear, the predicted position of the subject eye E is calculated from the positional relationship between the detected feature portion and the entire face image of the subject eye E. As the predicted position of the subject eye E, for example, when the central position of the anterior ocular segment image in which the subject eye E does not appear is set as a reference position (xo, yo) in a two-dimensional coordinate plane of the XY-axes, a predicted target position (xt, yt) that is a pupil predicted position of the subject eye E in the entire face image is calculated.

In Step S6, following the calculation of the predicted position of the subject eye in Step S5, adjustment movement to the calculated predicted position of the subject eye is performed, and the process proceeds to Step S2. Here, in the "adjustment movement to the predicted position of the subject eye E", the XYZ driver 18 is used for movement in the X-axis direction, and the XYZ driver 18 is used for movement in the Y-axis direction when the Y-axis movement amount is equal to or less than a predetermined amount and is small, and the chin rest driver 32 is used when the Y-axis movement amount exceeds the predetermined amount and is relatively large. Note that, when the chin rest 30 is moved up and down in the Y-axis direction using the chin rest driver 32, it is notified in advance that the chin rest 30 is moved upward or downward by a voice announcement to prevent the subject from being surprised or uncomfortable by sudden movement.

In Step S7, following the determination that the feature portion of the face has not been detected in Step S4, imaging is performed with the magnification of the anterior ocular segment stereo camera 22 lowered, the anterior ocular segment image is acquired again from the anterior ocular segment stereo camera 22, and the process proceeds to Step S2. That is, by imaging the anterior ocular segment stereo camera 22 with the lowered magnification, the angle of view of the anterior ocular segment image is enlarged as a whole as compared with the angle of view of the anterior ocular segment image with a normal high magnification, and the subject eye E can appear in the anterior ocular segment image if the subject puts his or her chin on the chin rest 30.

Next, the processing operation of the imaging start mode control when the anterior ocular segment imaging is started by the anterior ocular segment stereo camera 22 will be described with reference to FIG. 6.

When the anterior ocular segment is imaged in Step S1 and the subject eye E is detected in the anterior ocular segment image at the time of starting the imaging in Step S2, the process proceeds in the order of Step S 1, Step S2, Step S3 and end in the flowchart of FIG. 6. That is, when the subject eye E is detected in the anterior ocular segment image in Step S2, the process proceeds to Step S3, and in Step S3, the fine alignment control by the fine alignment controller 633 is executed following the execution of the coarse alignment control by the coarse alignment controller 632. Note that, after the alignment control is finished, for example, autofocus control on the eye fundus, or the like is executed.

On the other hand, when the anterior ocular segment is imaged in Step S1 and the subject eye E is not detected in the anterior ocular segment image at the time of starting imaging in next Step S2, the process proceeds in the order of Step S1, Step S2, and Step S4 in the flowchart of FIG. 6. In Step S4, it is determined whether or not the feature portion of the face is detected by the machine learning detection processing using the anterior ocular segment image in which the subject eye E does not appear and the learned feature portion detection model. When it is determined in Step S4 that the feature portion of the face is detected, the process proceeds from Step S4 to Step S5, and the predicted position of the subject eye E is calculated in Step S5. In Step S6, the adjustment movement to the calculated predicted position of the subject eye E is performed, and the process returns from Step S6 to Step S2. Then, in Step S2, it is determined whether or not the subject eye E has been detected in the anterior ocular segment image by the adjustment movement to the predicted position of the subject eye E.

While the subject eye E is not detected in the anterior ocular segment image regardless of the adjustment movement to the predicted position of the subject eye E in Step S2, the flow in the order of Step S2, Step S4, Step S5, and Step S6 is repeated. Then, when the subject eye E is detected in the anterior ocular segment image in Step S2 as the adjustment movement amount to the predicted position of the subject eye E increases, the adjustment movement to the predicted position of the subject eye E is stopped, and the process proceeds in the order of Step S2, Step S3, and end.

Further, when it is determined in Step S2 that the subject eye E is not detected in the anterior ocular segment image at the time of starting the imaging, and a feature portion of the face necessary for predicting the position of the subject eye E is not detected in Step S4, the process proceeds from Step S4 to Step S7. In Step S7, imaging is performed with the magnification of the anterior ocular segment stereo camera 22 lowered, and the anterior ocular segment image is acquired again from the anterior ocular segment stereo camera 22, and the process proceeds to Step S2. Then, in Step S2, when the subject eye E is detected in the anterior ocular segment image by imaging with the magnification of the anterior ocular segment stereo camera 22 set to a low magnification, the process proceeds in the order of Step S2, Step S3, and end.

(Imaging start mode control operation) The imaging start mode control operation will be described with reference to FIGS. 7 to 9. In the imaging start mode control by automatic alignment, the display screen 41 of the control panel 40 or the display screen 41' of the remote control tablet 40' becomes the imaging start auto-adjustment mode screen 43 illustrated in FIG. 7.

The imaging start auto-adjustment mode screen 43 includes a menu button 431, a patient ID display area 432, an imaging information display area 433, a first moving image area 434, a second moving image area 435, an in-process message display 436, and the capture start button 437. When the menu button 431 is tapped, the screen shifts to the imaging icon selection screen. The patient ID display area 432 displays a patient ID. The imaging information display area 433 displays information about the imaged eye and the angle of view. The first moving image area 434 displays an anterior ocular segment moving image from the first camera 22a of the anterior ocular segment stereo camera 22. The second moving image area 435 displays an anterior ocular segment moving image from the second camera 22b of the anterior ocular segment stereo camera 22. The in-process message display 436 displays a current process state ("under auto-alignment"). When the capture start button 437 is tapped, imaging or capturing is started. Note that the "patient ID" refers to a "subject ID".

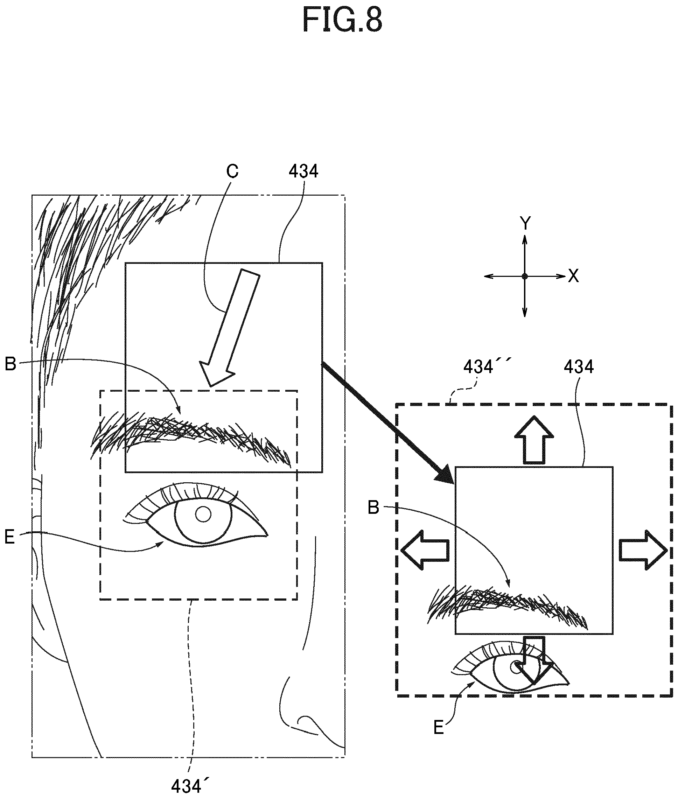

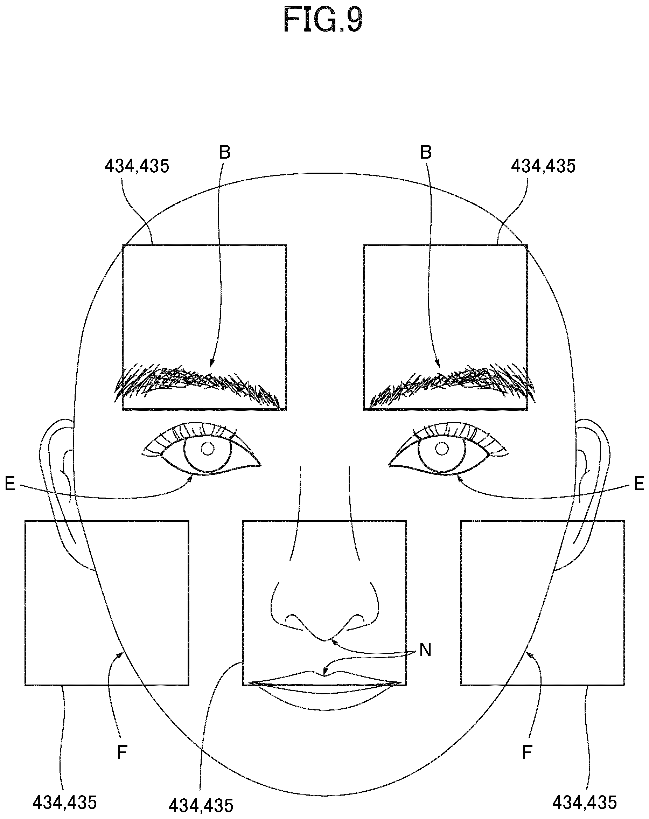

For example, in the anterior ocular segment image displayed in the first moving image area 434 and the anterior ocular segment image displayed in the second moving image area 435, as illustrated in FIG. 7, it is assumed that the subject eye E (pupil) does not appear in the anterior ocular segment images and the eyebrows B appear in the anterior ocular segment images. In this case, the eyebrows B, which are the features portion of the face, are detected from both anterior ocular segment images in the first moving image area 434 and the second moving image area 435. Then, as illustrated in the left part of FIG. 8, the predicted position of the subject eye E is calculated based on the positional relationship between the detected eyebrow B and the subject eye E in the entire face, and the adjustment movement to the calculated predicted position of the subject eye E is performed. When the adjustment movement in the X-axis direction and the Y-axis direction to the predicted position of the subject eye E is replaced with the movement of the anterior ocular segment image, the first moving image area 434 moves in the direction indicated by the arrow C in FIG. 8 and becomes a first moving image area 434'. Then, when the movement amount in the direction indicated by the arrow C reaches a predetermined amount by the adjustment movement to the predicted position of the subject eye E, the subject eye E (pupil) is detected in the anterior ocular segment image in the first moving image area 434'. Here, the feature portion of the face may be any part of the face other than the subject eye E, and is not limited to the eyebrow B, and may be a facial contour F, a nose, a mouth N, ears, and the like as illustrated in FIG. 9.

Further, when the subject eye E does not appear in the anterior ocular segment image and the feature portion of the face cannot be detected, the magnification of the anterior ocular segment stereo camera 22 is lowered, and the anterior ocular segment image is acquired again from the anterior ocular segment stereo camera 22. That is, by reducing the magnification of the anterior ocular segment stereo camera 22, the angle of view of the anterior ocular segment image displayed in the first moving image area 434 is enlarged as illustrated in the right part of FIG. 8 and becomes the angle of view of the first moving image area 434", and the subject eye E enters the angle of view of the anterior ocular segment image displayed in the first moving image area 434".

Meanwhile, the background art of alignment control in the ophthalmologic apparatus is executed at least when the condition that the subject eye E appears in the anterior ocular segment image is satisfied, and in a case where the subject eye E does not appear, it is not subject to the alignment control and is left to the examiner to handle. On the other hand, the present inventors have focused on the point that even in a situation where the subject eye E does not appear in the anterior ocular segment image, if the face part appearing in the anterior ocular segment image can be grasped by image recognition, the face part can be included in the target of alignment control.

That is, when it is determined that the subject eye E does not appear in the anterior ocular segment image in the imaging start mode of the anterior ocular segment by the anterior ocular segment stereo camera 22, the alignment controller 63 automatically calculates the predicted position of the subject eye E based on the image recognition of the face part appearing in the anterior ocular segment image following the determination. Then, a configuration including the imaging start mode controller 631 that performs control of moving toward the calculated predicted position of the subject eye E is employed.

Therefore, in a case where the subject eye E does not appear in the anterior ocular segment image in the imaging start mode of the anterior ocular segment, the XY-axes alignment adjustment to the position where the subject eye E appears can be automatically performed. As a result, preparation work that makes the subject eye E appear in the anterior ocular segment image by the examiner correcting an inclination of the face of the subject or the examiner performing an alignment adjustment by manual operation, and the like, becomes unnecessary, and thus the examiner's burden is reduced. Furthermore, when the examiner measures the eye characteristics by remote control at a position away from the subject or in another room, the examiner does not need to move to the position where the subject is located to perform preparation work, which reduces the number of work steps. In addition, with respect to Patent Literature 1, even in a case where there is no magnification changing means, XY-axes auto-alignment adjustment to a position where the subject eye E appears can be performed by position prediction of the subject eye E.

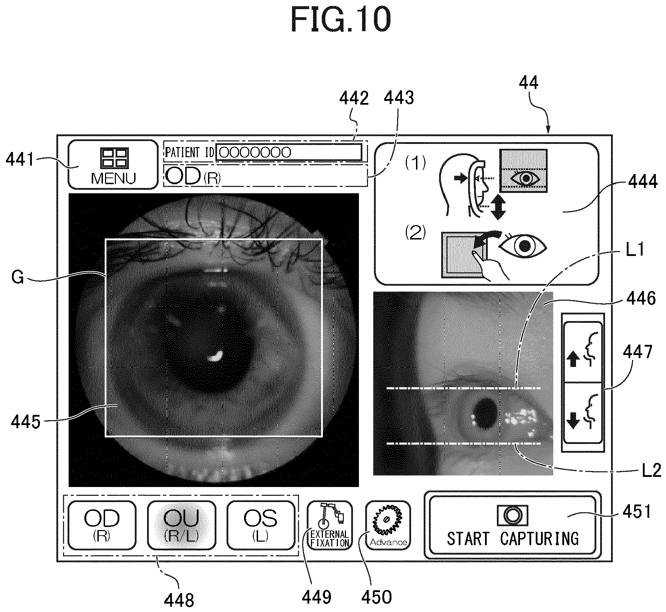

(Coarse alignment control operation) A coarse alignment control operation will be described with reference to FIG. 10. The coarse alignment control operation is executed following the imaging start mode control for starting the anterior ocular segment imaging by the anterior ocular segment stereo camera 22. The coarse alignment control is performed by the manual operation of the examiner based on a coarse manual adjustment mode screen 44 illustrated in FIG. 10 displayed on the control panel 40 or the remote control tablet 40'.

The coarse manual adjustment mode screen 44 includes a menu button 441, a patient ID display area 442, an imaging information display area 443, an operation method guide 444, a first moving image area 445, and a second moving image area 446. The coarse manual adjustment mode screen 44 further includes a chin rest vertical movement button 447, an imaged eye selection button 448, an external fixation button 449, an advance button 450, and a capture start button 451. Note that the operation method guide 444 displays an operation method on this screen. The first moving image area 445 displays the anterior ocular segment observation image from the measurement optical system 50. The second moving image area 446 displays an image of the anterior ocular segment stereo camera 22 closer to the imaged eye. The chin rest vertical movement button 447 vertically moves the chin rest 30 by a touch operation. The imaged eye selection button 448 selects an imaged eye. When tapped, the external fixation button 449 switches to the external fixation target 35, displays the ON/OFF state of the external fixation target 35, and switches ON/OFF of the external fixation target 35. The advance button 450 switches the mode to the advance mode when tapped.

The coarse alignment control is performed by Y-axis manual alignment adjustment using the anterior ocular segment image and XY-axes manual alignment adjustment using the anterior ocular segment observation image. The Y-axis manual alignment adjustment by the anterior ocular segment image is performed by manually operating the chin rest vertical movement button 447 so that the height mark 33a of the chin rest 30 matches the height of the subject eye E with the lines L1 and L2 displayed in the second moving image area 446 as a guide. The XY-axes manual alignment adjustment by the anterior ocular segment observation image is performed by performing a touch operation on the pupil of the subject eye E appearing in the anterior ocular segment observation image so that the pupil of the anterior ocular segment observation image displayed in the first moving image area 445 enters a frame G.

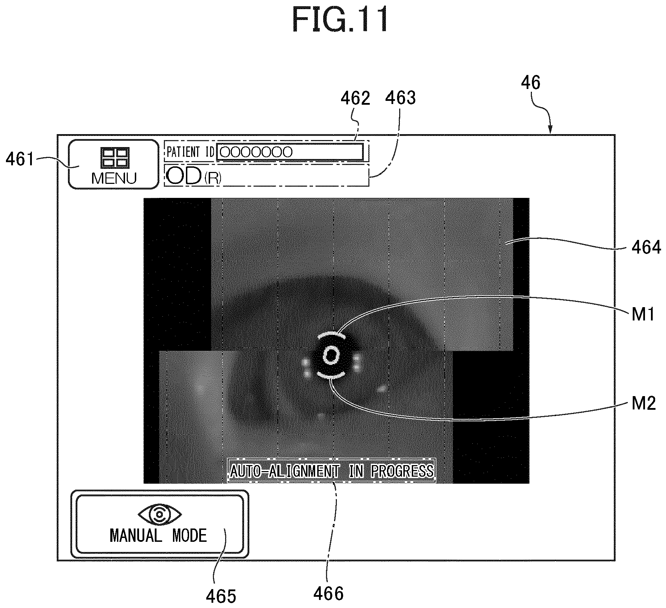

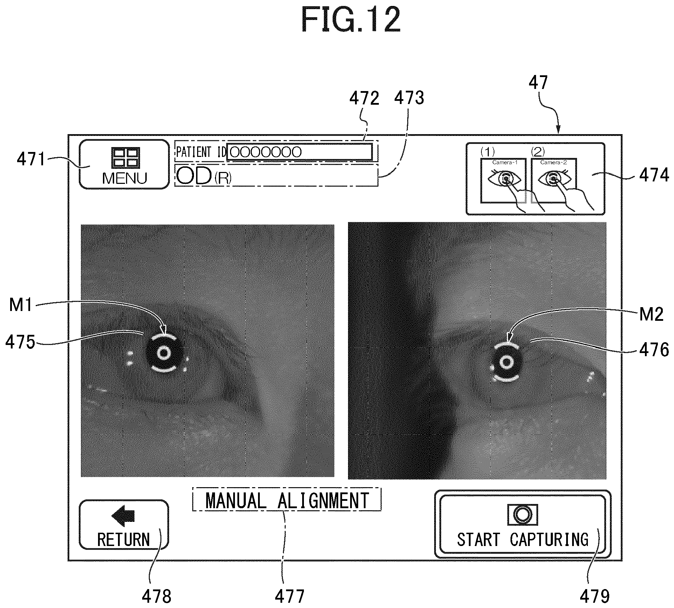

(Fine alignment control operation) A fine alignment control operation will be described with reference to FIGS. 11 and 12. The fine alignment control operation is performed after the coarse alignment control. The fine alignment control includes two types of alignment controls: fine auto-alignment control for performing auto-alignment with respect to the pupil and fine manual alignment control for performing manual alignment with respect to the pupil.

In the fine auto-alignment control, the display screen 41 of the control panel 40 or the display screen 41' of the remote control tablet 40' becomes a fine auto-adjustment mode screen 46 illustrated in FIG. 11. The fine auto-adjustment mode screen 46 includes a menu button 461, a patient ID display area 462, an imaging information display area 463, a moving image area 464, a manual mode button 465, and an in-process message 466. In the moving image area 464, the anterior ocular segment image from the first camera 22a and the anterior ocular segment image from the second camera 22b of the anterior ocular segment stereo camera 22 are displayed separately in an upper region and a lower region. When the manual mode button 465 is tapped, the automatic alignment adjustment is stopped, and the screen is switched to a fine manual adjustment mode screen 47 in which the imaged eye is manually adjusted. The in-process message 466 displays the state currently in process ("under auto-alignment"). The fine manual adjustment mode screen 47 will be described later.

In the fine auto-alignment control for the pupil, the XY-axes alignment adjustment is automatically performed by the XYZ driver 18 so that a pupil mark M1 in the upper region and a pupil mark M2 in the lower region displayed in the moving image area 464 coincide with each other. Note that the pupil marks M1 and M2 are marks indicating the pupil position of the anterior ocular segment image.

Next, the fine manual alignment control operation executed by tapping the manual mode button 465 in the fine auto-alignment control will be described with reference to FIG. 12. When the manual mode button 465 is tapped, the display screen 41 of the control panel 40 or the display screen 41' of the remote control tablet 40' is switched from the fine auto-adjustment mode screen 46 illustrated in FIG. 11 to the fine manual adjustment mode screen 47 illustrated in FIG. 12.

The fine manual adjustment mode screen 47 includes a menu button 471, a patient ID display area 472, an imaging information display area 473, an operation method guide 474, a first moving image area 475, and a second moving image area 476. Furthermore, the fine manual adjustment mode screen 47 includes an in-process message 477, a return button 478, and a capture start button 479. The operation method guide 474 displays an operation method of this screen. The first moving image area 475 displays the anterior ocular segment image from the first camera 22a of the anterior ocular segment stereo camera 22. The second moving image area 476 displays the anterior ocular segment image from the second camera 22b of the anterior ocular segment stereo camera 22. The in-process message 477 displays the current process state ("manual alignment"). When the return button 478 is tapped, the screen returns to the coarse manual adjustment mode screen 44.

In the fine manual alignment control for the pupil, the pupil mark M1 of the anterior ocular segment image displayed in the first moving image area 475 and the pupil mark M2 of the anterior ocular segment image displayed in the second moving image area 476 are tapped. Based on this tap operation, the XY-axes alignment adjustment is performed so that the pupil mark M1 and the pupil mark M2 are respectively arranged in the center of the anterior ocular segment image by the XYZ driver 18.

As described above, the ophthalmologic apparatus A of the first embodiment has the following effects. (1) An ophthalmologic apparatus A includes a main body 20, an anterior ocular segment camera (anterior ocular segment stereo camera 22), and a controller 60. The controller 60 includes an alignment controller 63 that performs control to adjust a relative positional relationship between a subject eye E and the main body 20 based on an anterior ocular segment image. The alignment controller 63 includes the imaging start mode controller 631 that, when it is determined that the subject eye E does not appear in the anterior ocular segment image in the imaging start mode of the anterior ocular segment by the anterior ocular segment camera, automatically calculates the predicted position of the subject eye E based on the image recognition of the face part appearing in the anterior ocular segment image following the determination, and performs control of moving toward the calculated predicted position of the subject eye E. Thus, when the subject eye E does not appear in the anterior ocular segment image in the imaging start mode of the anterior ocular segment, the alignment adjustment to the position where the subject eye E appears can be automatically performed.
(2) When the subject eye E is detected in the anterior ocular segment image from the anterior ocular segment camera (anterior ocular segment stereo camera 22) after starting the control of moving toward the predicted presence position of the subject eye E, the imaging start mode controller 631 stops the movement upon the detection and switches to the normal alignment control. Thus, when the subject eye E is detected in the anterior ocular segment image by the imaging start mode control without changing the existing normal alignment control, the imaging start mode control can be connected to the normal alignment control.
(3) When it is determined that the subject eye E does not appear in the anterior ocular segment image and the presence position of the subject eye E has not been predicted, the imaging start mode controller 631 decreases the magnification of the anterior ocular segment camera (anterior ocular segment stereo camera 22) and acquires the anterior ocular segment image from the anterior ocular segment camera again. Thus, when the subject eye E does not appear in the anterior ocular segment image and the presence position of the subject eye E has not been predicted, it is possible to increase the probability that the subject eye E is detected in the anterior ocular segment image by enlarging the angle of view of the anterior ocular segment image.
(4) When it is determined that the subject eye E does not appear in the anterior ocular segment image, the imaging start mode controller 631 detects a feature portion of the face from the anterior ocular segment image in which the subject eye E does not appear, and calculates a predicted position of the subject eye E from the positional relationship between the detected feature portion and the entire face image of the subject eye E. Thus, the predicted position of the subject eye E can be accurately calculated from the positional relationship between the detected feature portion and the entire face image of the subject eye E based on the detection of the feature portion of the face.
(5) The imaging start mode controller 631 includes a learned feature portion detection model constructed in advance by execution of machine learning using a large number of generated machine learning data sets of feature portion images and a selected machine learning model and detects the feature portion of the face based on image recognition using the anterior ocular segment image in which the subject eye E does not appear and the learned feature portion detection model. Thus, by the image recognition method using the learned feature portion detection model, the feature portion of the face can be accurately detected from the anterior ocular segment image in which the subject eye E does not appear.

### Second Embodiment

The second embodiment is an example in which the feature portions of the face are individually detected by image recognition, and the imaging start mode control is performed by setting the detection order such that detection of the eyebrow B is prioritized over the detection of the contour F and the detection of the nose and the mouth N. Note that the configuration of the ophthalmologic apparatus A of the second embodiment is similar to the configuration of the first embodiment illustrated in FIGS. 1 to 5, and thus illustration and description of the similar configuration are omitted.

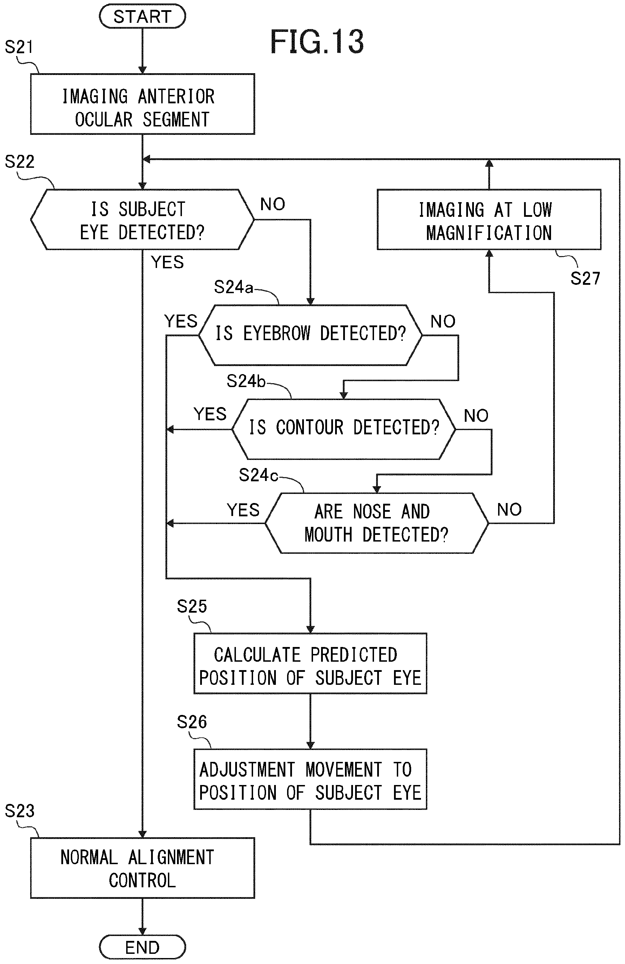

(Processing configuration and processing operation of imaging start mode control) A processing configuration of the imaging start mode control executed by the imaging start mode controller 631 of the second embodiment will be described with reference to a flowchart illustrated in FIG. 13. Note that Steps S21, S22, S23, S25, S26, and S27 in FIG. 13 perform the same processing as Steps S 1, S2, S3, S5, S6, and S7 in FIG. 6, and thus descriptions of above Steps in FIG. 13 are omitted.

In Step S24a, following the determination in Step S22 that the subject eye is not detected, it is determined whether or not an eyebrow B is detected by the machine learning detection processing using the anterior ocular segment image in which the subject eye E does not appear and a learned eyebrow detection model (learned individual portion detection model). When the eyebrow B is detected (i.e., YES) in Step S24a, the process proceeds to Step S25. On the other hand, when the eyebrow B is not detected (i.e., NO) in Step S24a, the process proceeds to Step S24b. That is, when the subject eye E does not appear in the anterior ocular segment image, the highest priority is given to the detection of the eyebrow B from the anterior ocular segment image in which the subject eye E does not appear using the learned eyebrow detection model. Then, the detection order of the individual feature portions is defined in which the contour F is detected when the eyebrow B is not detected, and the nose and the mouth N are detected when the contour F is not detected.

Here, the "learned individual portion detection model" refers to a model constructed in advance by executing machine learning using a large number of machine learning data sets and the selected machine learning model. In the machine learning data sets, the feature portions are divided into individual portions of individual portions of the individual portions into the eyebrow B, the contour F, and the nose and mouth N, and the machine learning data sets are generated for each of the individual portions. That is, as the "learned individual portion detection model", "learned eyebrow detection model", "learned contour detection model", and "learned nose and mouth detection model" specialized for the detection of the individual portions of the eyebrow B, the contour F, and the nose and mouth N are prepared in advance (see FIG. 9).

In Step S24b, following the non-detection of the eyebrow B in Step S24a, it is determined whether or not the contour F is detected by the machine learning detection processing using the anterior ocular segment image in which the subject eye E does not appear and the learned contour detection model. When the contour F is detected (i.e., YES) in Step S24b, the process proceeds to Step S25. On the other hand, when the contour F is not detected (i.e., NO) in Step S24b, the process proceeds to Step S24c.

In Step S24c, following the non-detection of the contour F in Step S24b, it is determined whether or not the nose and the mouth N are detected by the machine learning detection processing using the anterior ocular segment image in which the subject eye E does not appear and the learned nose and mouth detection model. When the nose and mouth N are detected (i.e., YES) in Step S24c, the process proceeds to Step S25. On the other hand, when the nose and mouth N are not detected (i.e., NO) in Step S24c, the process proceeds to Step S27.

Next, a processing operation of the imaging start mode control when the anterior ocular segment imaging is started by the anterior ocular segment stereo camera 22 will be described with reference to FIG. 13.

When the anterior ocular segment is captured or imaged in Step S21 and the subject eye E is detected in the anterior ocular segment image at the time of starting imaging in Step S22, the process proceeds in the order of Step S21, Step S22, Step S23, and end in the flowchart of FIG. 13. That is, when the subject eye E is detected in the anterior ocular segment image in Step S22, the process proceeds to Step S23, and in Step S23, the fine alignment control by the fine alignment controller 633 is executed following the execution of the coarse alignment control by the coarse alignment controller 632.

On the other hand, when the anterior ocular segment is captured or imaged in Step S21 and the subject eye E is not detected in the anterior ocular segment image at the time of starting the imaging in Step S22, the process proceeds in the order of Step S21, Step S22, and Step S24a in the flowchart of FIG. 13. In Step S24a, it is determined whether or not the eyebrow B is detected by the machine learning detection processing using the anterior ocular segment image in which the subject eye E does not appear and the learned eyebrow detection model. When it is determined in Step S24a that the eyebrow B is detected, the process proceeds from Step S24a to Step S25, and in Step S25, a predicted position of the subject eye E is calculated. In the next Step S26, the adjustment movement to the calculated predicted position of the subject eye E is performed, and the process returns to Step S22 from Step S26. In Step S22, it is determined whether or not the subject eye E has been detected in the anterior ocular segment image by the adjustment movement to the predicted position of the subject eye E. Then, when the subject eye E is detected in the anterior ocular segment image in Step S22, the adjustment movement to the predicted position of the subject eye E is stopped, and the process proceeds in the order of Step S22, Step S23, and end.

Next, when it is determined in Step S22 that the subject eye E is not detected in the anterior ocular segment image at the imaging start point and the eyebrow B is not detected in Step S24a, the process proceeds from Step S24a to Step S24b. In Step S24b, it is determined whether or not the contour F has been detected by the machine learning detection processing using the anterior ocular segment image in which the subject eye E does not appear and the learned contour detection model. When it is determined in Step S24b that the contour F has been detected, the process proceeds from Step S24b to Step S25, and in Step S25, a predicted position of the subject eye E is calculated. In the next Step S26, the adjustment movement to the calculated predicted position of the subject eye E is performed, and the process returns to Step S22 from Step S26. In Step S22, it is determined whether or not the subject eye E has been detected in the anterior ocular segment image by the adjustment movement to the predicted position of the subject eye E. Then, when the subject eye E is detected in the anterior ocular segment image in Step S22, the adjustment movement to the predicted position of the subject eye E is stopped, and the process proceeds in the order of Step S22, Step S23, and end.

Next, when it is determined in Step S22 that the subject eye E is not detected in the anterior ocular segment image at the imaging start point and the contour F is not detected in Step S24b, the process proceeds from Step S24b to Step S24c. In Step S24c, it is determined whether or not the nose and the mouth N have been detected by the machine learning detection processing using the anterior ocular segment image in which the subject eye E does not appear and the learned nose and mouth detection model. When it is determined that the nose and the mouth N are detected in Step S24c, the process proceeds from Step S24c to Step S25. In Step S25, a predicted position of the subject eye E is calculated. In the next Step S26, the adjustment movement to the calculated predicted position of the subject eye E is performed, and the process returns to Step S22 from Step S26 In Step S22, it is determined whether or not the subject eye E has been detected in the anterior ocular segment image by the adjustment movement to the predicted position of the subject eye E. Then, when the subject eye E is detected in the anterior ocular segment image in Step S22, the adjustment movement to the predicted position of the subject eye E is stopped, and the process proceeds in the order of Step S22, Step S23, and end.

Next, when it is determined in Step S24c that the nose and the mouth N are not detected, the process proceeds from Step S24c to Step S27. In Step S27, imaging is performed with the magnification of the anterior ocular segment stereo camera 22 lowered, and the anterior ocular segment image is acquired again from the anterior ocular segment stereo camera 22, and the process proceeds to Step S22. Then, in Step S22, when the subject eye E is detected in the anterior ocular segment image by imaging with the magnification of the anterior ocular segment stereo camera 22 set to the low magnification, the process proceeds in the order of Step S22, Step S23, and end.

(Imaging start mode control operation) In the second embodiment, the feature portions of the face are individually detected by image recognition, and the detection order is set such that the detection of the eyebrow B is prioritized over the detections of the contour F and the nose and mouth N. This is based on the fact that the inventors have found that the eyebrow B is the most frequently detected among the feature portions of the face when the subject eye E is not detected in the anterior ocular segment image.

That is, when the face of the subject does not face the front but is inclined, the first subject eye E which is higher than the second subject eye E is detected in the anterior ocular segment image while the second subject eye E which is lower than the first subject eye E is not detected in the anterior ocular segment image but the eyebrow B is detected. In addition, when the previous subject is an adult and the current subject is a child, if the subject is switched to the child in a state where the chin rest 30 is set for the adult at the height position in the Y-axis direction at which the left and right subject eyes E are detected in the anterior ocular segment image with the adult face, the left and right eyebrows B of the child subject are detected in the anterior ocular segment image.

Therefore, when the detection order is set such that the detection of the eyebrow B is prioritized over the detections of the contour F and the nose and mouth N, the eyebrow B can be detected earlier than in a case where one of a plurality of feature portions in the face is extracted and detected by image recognition. Then, when the eyebrow B is detected earlier, the time required to complete the imaging start mode control through the calculation of the predicted position of the subject eye E and the adjustment movement to the predicted position after detecting the eyebrow B can be shortened.

As described above, the ophthalmologic apparatus A of the second embodiment has the following effects in addition to the effects of (1) to (4) of the first embodiment.
(6) The imaging start mode controller 631 includes a learned individual portion detection model for the eyebrow B, the contour F, the nose, and the mouth N constructed in advance by execution of the machine learning using a large number of generated machine learning data sets of the feature portion images and a selected machine learning model. When the feature portion of the face is detected based on the image recognition using the anterior ocular segment image in which the subject eye E does not appear and the learned individual portion detection model, the order for each of the feature portions is set such that the detection of the eyebrow B is prioritized over the detections of the contour F and the nose and mouth N. Thus, by the image recognition method using the learned individual portion detection model, the individual portion of the face can be accurately detected from the anterior ocular segment image in which the subject eye E does not appear, and the time required for the imaging start mode control can be shortened by prioritizing the detection of the eyebrow B with high frequency.

The ophthalmologic apparatus of the present invention has been described above based on the first and second embodiments. However, the specific configuration of the ophthalmologic apparatus is not limited to these embodiments, and design changes, additions, and the like are allowed without departing from the gist of the invention according to each of the claims.

In the first and second embodiments, the example has been described in which, as the imaging start mode controller 631, when it is determined that the subject eye E does not appear in the anterior ocular segment image in the imaging start mode of the anterior ocular segment by the anterior ocular segment stereo camera 22, the alignment control based on the image recognition of the face part is automatically performed following the determination. However, as the imaging start mode controller, an example may be applicable in which when it is determined that the subject eye does not appear in the anterior ocular segment image in the imaging start mode of the anterior ocular segment by the anterior ocular segment camera, the alignment control based on the image recognition of the face part may be automatically performed after performing at least notification following the determination. That is, following the determination that the subject eye does not appear, the alignment control based on image recognition of the face part may be automatically performed after the notification. Alternatively, following the determination that the subject eye does not appear, the notification may be performed, and the alignment control based on the image recognition of the face part may be automatically performed after receiving a predetermined operation by the examiner. Here, the "notification" means notifying the subject in advance by an announcement or the like that the main body or the chin rest moves by execution of the alignment control.

In the first and second embodiments, the example has been described in which, as the imaging start mode controller 631, when the subject eye E is detected in the anterior ocular segment image from the anterior ocular segment stereo camera 22 after starting the control of moving toward the predicted presence position of the subject eye E, the movement is stopped upon the detection, and it is switched to the normal alignment control. However, an example may be given in which, as the imaging start mode controller, after the control of moving to the predicted presence position of the subject eye is terminated, it is confirmed that the subject eye is detected in the anterior ocular segment image, and it is switched to the normal alignment control.

In the first embodiment, the low magnification imaging is performed when the feature portion of the face has not been detected. In the second embodiment, the process proceeds to the low magnification imaging when none of the eyebrow B, the contour F, and the nose and the mouth N has been detected. However, in the first and second embodiments, "when the presence position of the subject eye E has not been predicted" may include a case where the feature portion of the face has been detected but the predicted position of the subject eye has not been calculated.

In the first embodiment, as the imaging start mode controller 631, the example has been described in which the feature portion of the face is detected based on the image recognition using the anterior ocular segment image in which the subject eye E does not appear and the learned feature portion detection model. In the second embodiment, as the imaging start mode controller 631, the example has been described in which the eyebrow B, the contour F, the nose, and the mouth N are individually detected based on the image recognition using the anterior ocular segment image in which the subject eye E does not appear and the learned individual portion detection model. However, as the imaging start mode controller, an example is applicable in which the feature portion of the face or the individual portion of the face may be detected by a method such as pattern matching or landmark matching without using the learned feature portion detection model or the learned individual portion detection model by the machine learning. Here, the "pattern matching" refers to a method of preparing a pattern map in which a feature portion is digitized in advance by a shape pattern, and performing matching determination between the shape pattern acquired by image processing of the anterior ocular segment image in which the subject eye does not appear and the pattern map to specify the feature portion. Note that, in addition to the shape pattern, pattern matching may be performed including a color pattern and a luminance pattern. The "landmark matching" refers to a method in which landmarks (numbered feature points) of the face, which are important key points in detecting features such as the positions of the eyes and nose, are determined, and the feature portion is specified by the number of the landmarks acquired from the anterior ocular segment image in which the subject eye does not appear.

In the first and second embodiments, as the imaging start mode controller 631, an example has been described in which the imaging start mode control process is started by a predetermined operation after it is confirmed that the subject is seated in front of the ophthalmologic apparatus A with the power switch turned on and the chin is supported by the chin rest 30. However, an example may be given in which, as the imaging start mode controller, the imaging start mode control process is started when it is detected by a sensor or a switch that the subject places his or her chin on the chin rest and rests his or her forehead.

In the first and second embodiments, an example in which the anterior ocular segment stereo camera 22 is used as the anterior ocular segment camera has been described. However, the anterior ocular segment camera is not limited to the stereo camera and may be, for example, a monocular camera. In addition, a camera that does not have a zoom function for changing magnification may be used.

In the first and second embodiments, as the ophthalmologic apparatus, an application example to the ophthalmologic apparatus A in which the anterior ocular segment image of the subject eye, the fundus image of the subject eye, and the fundus tomographic image of the subject eye are observed, imaged, and recorded, and provided as the electronic image for diagnosis has been described. However, the ophthalmologic apparatus is not limited to the application to the ophthalmologic apparatus A. That is, the alignment control technique of the present invention can be applied to any ophthalmologic apparatus that requires alignment control for adjusting the relative positional relationship between the subject eye and the apparatus main body, regardless of whether the ophthalmologic apparatus is a subjective ophthalmologic apparatus or an objective ophthalmologic apparatus.

### List of Reference Signs

- A: OPHTHALMOLOGIC APPARATUS
- 18: XYZ DRIVER
- 20: MAIN BODY
- 22: ANTERIOR OCULAR SEGMENT STEREO CAMERA (ANTERIOR OCULAR SEGMENT CAMERA)
- 22A: FIRST CAMERA
- 22B: SECOND CAMERA
- 30: CHIN REST PORTION
- 32: CHIN REST DRIVER
- 50: MEASUREMENT OPTICAL SYSTEM
- 60: CONTROLLER
- 63: ALIGNMENT CONTROLLER
- 631: IMAGING START MODE CONTROLLER
- E: SUBJECT EYE

## Claims

1. An ophthalmologic apparatus (A) comprising:
a main body (20) comprising a measurement optical system (50) that is configured to measure eye characteristics of a subject eye (E) of a subject in a state where the subject has placed his or her chin on a chin rest (30);
an anterior ocular segment camera (22) that is provided in the main body (20), the anterior ocular segment camera (22) being configured to acquire an anterior ocular segment image by imaging an anterior ocular segment of the subject; and
a controller (60) that is configured to control each element of the apparatus;
wherein the controller (60) comprises an alignment controller (63) that is configured to perform control to adjust a relative positional relationship between the subject eye (E) and the main body (20) based on the anterior ocular segment image, and
wherein the alignment controller (63) comprises an imaging start mode controller (631), the imaging start mode controller (631) being configured, when it is determined that the subject eye (E) does not appear in the anterior ocular segment image in an imaging start mode of the anterior ocular segment by the anterior ocular segment camera (22), to calculate a predicted position of the subject eye (E) based on image recognition of a face part of the subject in the anterior ocular segment image automatically following the determination or automatically after performing at least notification following the determination, and to perform control of movement toward the calculated predicted position of the subject eye (E).

2. The ophthalmologic apparatus (A) according to claim 1,
wherein the imaging start mode controller (631) is configured, when the subject eye (E) is detected in the anterior ocular segment image from the anterior ocular segment camera (22) after starting the control of movement toward a predicted presence position of the subject eye (E), to stop the movement at a stage of the detection and to switch to normal alignment control.

3. The ophthalmologic apparatus (A) according to claim 1,
wherein the imaging start mode controller (631) is configured, when it is determined that the subject eye (E) does not appear in the anterior ocular segment image and a presence position of the subject eye (E) cannot be predicted, to decrease magnification of the anterior ocular segment camera (22) and to acquire the anterior ocular segment image from the anterior ocular segment camera (22) again.

4. The ophthalmologic apparatus (A) according to any one of claims 1 to 3,
wherein the imaging start mode controller (631) is configured, when it is determined that the subject eye (E) does not appear in the anterior ocular segment image, to detect a feature portion of a face of the subject from the anterior ocular segment image in which the subject eye (E) does not appear and to calculate a predicted position of the subject eye (E) from a positional relationship between the detected feature portion and the subject eye (E) in an entire face.

5. The ophthalmologic apparatus (A) according to claim 4,
wherein the imaging start mode controller (631) comprises a learned feature portion detection model that is constructed in advance by execution of machine learning using a large number of generated machine learning data sets of feature portion images and a selected machine learning model, and
wherein the imaging start mode controller (631) is configured to detect a feature portion of the face based on image recognition using the anterior ocular segment image in which the subject eye (E) does not appear and the learned feature portion detection model.

6. The ophthalmologic apparatus (A) according to claim 4,
wherein the imaging start mode controller (631) comprises a learned individual portion detection model for an eyebrow (B), a contour (F), a nose, and a mouth (N) constructed in advance by execution of machine learning using a large number of generated machine learning data sets of feature portion images and a selected machine learning model, and
wherein a detection order for each of the feature portions is set such that detection of the eyebrow (B) is prioritized over detection of the contour (F) and detection of the nose and the mouth (N) when the feature portion of the face is detected based on the image recognition using the anterior ocular segment image in which the subject eye (E) does not appear and the learned individual portion detection model.
